# NEW EUROPEAN PATENT SPECIFICATION

(11) **EP 0 718 628 B2**
(45) Date of publication and mention of the opposition decision: **23.02.2005**
(45) Mention of the grant of the patent: 18.04.2001
(21) Application number: 95304016.9
(22) Date of filing: 09.06.1995
(51) Int. Cl.: G01N 33/86, C12Q 1/56

(54) **Method for diagnosis of blood coagulation disorders**
Verfahren zur Diagnose von Blutgerinnungsstörungen
Procédé de diagnostic de désordres de la coagulation du sang

(30) Priority: 23.12.1994 EP 94309764
(43) Date of publication of application: 26.06.1996
(73) Proprietor: Diagnostic Reagents Limited, Thame, Oxon OX9 3NY (GB)
(72) Inventor: Denson, Kenneth William Ernest, Emmington, Oxon OX9 4AA (GB)
(74) Representative: Marlow, Nicholas Simon

(56) References cited:
- EP-A- 0 069 642
- EP-A- 0 236 985
- EP-A- 0 711 838
- WO-A-93/10261
- WO-A-94/17415
- DE-A- 4 427 785
- DE-A- 4 440 097
- DE-A- 19 506 236
- DAHLBAECK ET AL: 'Familial thrombophilia due to a ....' PROCEEDINGS OF THE NATIONAL ACADEMY OF SCIENCE vol. 90, WASHINGTON DC, pages 1004 - 1008, XP002976876
- JORQUERA ET AL: 'Modified test for activated protein C resistance' THE LANCET vol. 344, 22 October 1994, LONDON, pages 1162 - 1163, XP000918443
- SHEN ET AL: 'Factor V and protein S as synergistic cofactors ....' THE JOURNAL OF BIOLOGICAL CHEMISTRY vol. 269, no. 29, 22 July 1994, pages 18735 - 18738, XP002004743
- K Stocker et al. (1988) Folia Hematol.8, 260-264
- M Kraus & C Wagner (1994) Thrombosis Res.76, 231-236
- T Koster et al (1993) Lancet 342, 1503-1506
- S Rosén et al (1994) J Int Soc Thromb Haemost 72, 255-260

## Description

The present invention relates to a novel method useful for screening and diagnosis of thromboembolic diseases, such as hereditary thrombophilia. The invention can also be used for determining the risk for thrombosis in pregnant women, patients undergoing surgery, and subjects taking contraceptive drugs.

Blood coagulation comprises a complex system of interlinked proenzymes, enzymes and cofactors which react at the surfaces of activated platelets and endothelial cells. When the system is activated the ultimate result is the formation of a blood clot containing insoluble fibrin. The initiation and termination of fibrin formation is carefully regulated in normal homeostasis. In vitro, platelets and endothelial cell surfaces are usually substituted with suitable phospholipids. For the invention the relevant part of the coagulation system is that shown below.

Tissue thromboplastin
FVII
Ca++
FIX
FXIa
Ca++
FX
FIXa
Phospholipid
Ca++
Prothrombin
FVIII
FVIIIa
FXa
Phospholipid
Ca++
Protein S
Activated
Protein C
(APC)
FV
Cofactor
FVa
FXIII
Thrombin
FXIIIa
Thrombomodulin
Protein C
Fibrinogen
Fibrin
(soluble)
Fibrin
(insoluble)
procoagulant step
inhibition

Protein C is a zymogen which in vitro may be activated by thrombin (Factor IIa) alone, the combination thrombin-thrombomodulin, a fraction from certain snake venoms, such as Agkistrodon Contortrix Contortrix, or purified Factor Xa. Activation of plasma sample endogenous Protein C or addition of exogenously activated Protein C to a plasma sample in the presence of Protein S (PS) and Activated Protein C (APC) cofactor, will neutralise Factors Va and VIIIa and lead to prolonged blood coagulation clotting times in the plasma samples of healthy individuals. Factors V and VIII are activated by small amounts of thrombin or Factor Xa. Protein S is a cofactor to Protein C. Hereditary homozygous or heterozygous deficiencies in Protein C, Protein S and Antithrombin III (ATIII, an inhibitor of Factors XIa, IXa, Xa and Thrombin) are found in approximately 10-15% of patients with diagnosed thromboembolic disease before the age of 40. Homozygous Protein C and S deficiencies are life threatening and affected individuals develop generalised microvascular thrombosis and purpura fulminans in the neonatal period.

Deficiencies in Protein C and S and ATIII are measured by both functional and immunological methods. One way of determining functional Protein C activity involves the steps of mixing the plasma sample to be tested with an excess of human plasma deficient in Protein C, adding a Protein C activator and monitoring the appropriate substrate conversion. Alternatively substrates for enzymes such as thrombin and Factor Xa have been utilised; their activities are influenced by activated Protein C activity (i.e. enzyme activities that are generated or modulated by APC). In certain cases Protein C assays have involved isolation of the zymogen and a subsequent activation and addition of substrate to the activated form.

In order to determine Protein S functional activity in a plasma sample the most common methods involve mixing the plasma sample with activated Protein C, an excess of Protein S deficient plasma and further reagents necessary to achieve clotting (Waart et al., Thromb. Res. 48, 427-37 (1987); Suzuki et al., Thromb. Res. 49, 241-51 (1988); Bertina et al., Thromb. Haemost. 53,268-72 (1985); and Comp et al., J. Clin. Invest. 74, 2084-88 (1984)). It has also been suggested to measure Protein S by incubating the plasma sample with Factor IXa and activated Protein C and measurement of the clot time or the conversion of a chromogenic thrombin substrate (WO-A-9101382).

In addition to Protein C and Protein S abnormalities it has been shown (Dahlback B, Carlsson M and Svensson BJ. Proc. Natl Acad Sci 1993; 90:1004-08) that the commonest abnormality in hereditary thrombophilia involves the APC cofactor.

This cofactor is part of the accelerator Factor V molecule, which with APC forms an inhibitor which destroys Factor Va and Factor VIIIa, thus regulating the coagulation mechanism and preventing thrombosis. The abnormality in the Factor V molecule which predisposes to thrombosis is commonly due to a transitional mutation resulting in substitution of Arg⁵⁰⁶ to Gln⁵⁰⁶, known as Factor V Leiden.

In the paper previously cited by Dahlback et al a specific in vitro test for the detection of the APC cofactor in human plasma samples was described, as follows:

A plasma sample obtained from the subject was incubated for 5 minutes with
(a) an exogenous reagent (1) activating the intrinsic clotting mechanism followed by addition of calcium chloride (2) to complete the coagulation reaction; and separately with
(b) the same exogenous reagent (1) in which the coagulation reaction was completed by addition of calcium chloride containing an exogenous preparation of APC (3).
Step (a) is known as an ordinary Activated Partial Thromboplastin Time (APTT). Step (b) is an APTT with the addition of APC. The latter by-passes the inhibitory mechanism involving Protein C and Protein S. The clotting times of a sample in step (a) will vary between 25 seconds and 45 seconds depending upon the type of exogenous activating agent (APTT reagent). In the presence of normal APC cofactor the clotting time in step (b) will be prolonged. Dahlback expressed this as a ratio of b/a, that is an APC.APTT/APTT ratio. In normal persons the ratio is 2.0 to 4.0 and in patients with heterozygous or homozygous defects in the APC cofactor the ratio is less than 2.0. Thus for the diagnosis of the various defects in thrombophilia involving Protein C it is necessary to perform three separate tests; a Protein C assay, Protein S assay and an APC cofactor assay. The APC cofactor test alone will not detect the presence or absence of PS and PC.

In order to measure the APC.APTT/APTT ratio correctly in patients receiving treatment for thrombophilia, it has been proposed, for example by Jorquera et al, Lancet 1994, vol. 344, p.1162-3, to dilute the plasma sample 1:5 in Factor V depleted plasma.

Protein C may be activated by a fraction of A. Contortrix Contortrix snake venom, known as a Protein C activator (PCA), and when PCA is added to normal plasma, the inhibitor mechanism involving PC, PS and APC cofactor is initiated by the endogenous PC, PS and APC cofactor. WO 94/17415 discloses a test for APC activity which is similar to the test for APC cofactor described above from the paper by Dahlback *et al,* except that a PCA-calcium chloride mix is added to complete the coagulation reaction instead of an APC-calcium chloride mix. EP 0 236 985 discloses a spectrophotometric method for determination of protein C or protein S activity in a plasma sample based on measurements of the amount of thrombin produced upon activation of endogenous protein C with an exogenous PCA.

The present invention provides a test for the presence or absence of APC cofactor in human plasma samples in which PC and PS levels are normal by assay. It will also detect homozygous PC and PS defects and most, but not all, heterozygous defects in PC. The samples assayed are human plasma.

The invention provides an in vitro screening method for the exclusion of APC cofactor disorders as a cause of thrombophilia in a subject, such as a human subject, or for determining the risk of acquiring thrombophilia in a subject.

According to the invention there is provided an APC cofactor deficiency test for determining whether a blood plasma sample in which protein C and protein S levels are normal by assay is deficient in APC cofactor, comprising:
(i) incubating a first portion of the sample, preferably at 37°C, with
   a) an exogenous reagent containing phospholipid which activates the contact mechanism in intrinsic clotting an APTT reagent) or an exogenous reagent which initiates extrinsic clotting (a prothrombin time (PT) reagent); and incubating a second portion of the sample with
   b) the reagent of step (i) (a) and an exogenous Protein C (PC) activator which produces activated PC (APC) from endogenous Protein C and Protein S;
then (ii) adding to each of the two incubated portions a clotting agent, such as a divalent or trivalent salt, preferably calcium chloride, and monitoring the conversion rate of fibrinogen to insoluble fibrin for example manually or with a mechanical, photo-electrical or nephelometric instrument; and
(iii) calculating the ratio of the rate forthe second portion of the sample to the rate forthe first portion and comparing this ratio with the corresponding ratio for samples from normal subjects.
The ratio is referred to as the PCA.APTT/APTT ratio or the PCA.PT/PT ratio.

The invention is an in vitro method used as a screening test to eliminate defects in APC cofactor which could result in thrombophilia. Defects in APC cofactor are likely to occur in less than 5% of the normal population. The method of the invention is believed to exhibit greater discrimination than prior art tests, such as the APC cofactor test.

We have found that the PCA.APTT ratio for normal individuals is above 2.0 and may be as high as 10.0, while PCA.APTT ratios for APC cofactor defects are between 2.0 and 1.0. These figures may vary slightly for different APTT reagents and different instruments, and the lower limit of normality may be a ratio of 2.2 to 2.5.

The invention also provides a more definitive test for APC resistance. A potential problem with the APC cofactor test is that patients with thrombophilia may receive anticoagulant therapy, which leads to a reduction in Factors II, IX and X. This results in the APC cofactor test described above showing a higher PCA.APTT/APTT or PCA.PT/PT ratio than should be shown, leading to potential misdiagnosis of APC resistant patients as normal. According to this second aspect of the invention, Factors II, IX and X are normalised in the plasma sample (which may be APC resistant or sensitive) by diluting it with Factor V depleted plasma. Further, dilution in Factor V normalises levels of PC and PS in the diluted sample, irrespective of the levels in the undiluted sample. Preferably, the plasma is diluted 1:5 or 1:10 in the Factor V depleted plasma. The PCA.APTT/APTT or PCA.PT/PT ratio is then determined in accordance with the broad aspect of the invention described above.

The APC cofactor and Factor V coagulant activity are both part of the Factor V molecule and are therefore absent from Factor V depleted plasma. The dilution therefore maintains the balance in the plasma sample between the coagulant Factor V and the anticoagulant APC cofactor.

The intrinsic clotting Factors and PC and PS are at approximately the same level in any diluted sample; thus, the range of PCA.APTT/APTT or PCA.PT/PT values observed in plasma samples from normal patients is narrower than the corresponding range in tests using undiluted plasma.

The APTT exogenous reagent used in step (i) (a) may include micronised silica (preferably at about 0.06% in water and buffer), kaolin (preferably at from 0.25 to 10 mg/ml), bentonite, ellagic acid or any substance which activates the contact mechanism of intrinsic clotting, or any commercial liquid or lyophilised APTT reagent. The phospholipid may be a platelet substitute such as a brain extract or a relatively pure phospholipid.

The exogenous PT reagent used in step (i) (a) concerned in the extrinsic pathway may be bovine brain thromboplastin or any other organ species which gives a prolonged clotting time with normal plasma in the presence of PCA. Preferably, the PT reagent is the supernatant after centrifuging, preferably at about 500 g, a 4% solution of acetone-dried bovine brain activated in saline solution. Human or rabbit brain thromboplastin are unsatisfactory.

The Protein C activator used in step (i) (b) may be derived from human or animal components or fractions of snake venoms or any other exogenous activator of Protein C. The PCA used in step (i) (b) is combined with exogenous activator either in a liquid or lyophilised form. We have used a fraction of A. Contortrix Contortrix (Exner T and Vaasjoki R. Thromb. Haemost. 59:40-44 (1988)). The preferred concentration of PC activator is from 0.25.10⁻⁸M to 2.5.10⁻⁸M, most preferably 1.25.10⁻⁸M.

The optimum dilution in the APTT or PT reagent is that which gives the maximum prolongation in clotting time of pooled normal plasma in step (ii) for the product of step (i) (b). Higher or lower concentration may be used but in both cases clotting times are shorter, PCA ratios are lower and less sensitivity results.

Preferably, the reagents are in lyophilised form and are reconstituted with water or other diluent.

The calcium chloride in step (ii) is optionally 25mM but concentration from 10mM to 50mM may be used. Concentrations above 25mM may be inhibitory and concentrations below 10mM may be insufficient for clotting to take place. Magnesium and other divalent and trivalent cations may be incorporated.

In the prior art, saline is used in the preparation of the portion of the sample from which the APC.APTT time is measured. The use of saline in both APTT and PCA.APTT measurements is preferred to ensure that the clotting times observed are properly comparable. Preferably, the concentration of the saline is between 0.075M and 0.15M.

Preferably the exogenous reagent contains from 0.01 to 0.2%, preferably 0.04%, crude phospholipid. The optimum incubation time in (i) is 5 to 10 minutes for both APTT and PT reagents but times as short as 2 minutes or as long as 15 minutes may be employed. Longer incubation times may lead to deterioration of components in the plasma sample.

Also according to the invention there is provided a kit for carrying out the method of the invention comprising an APTT reagent or a PT reagent and, separately, the same reagent with a PC activator. The kit comprises further a clotting agent, preferably a divalent or trivalent salt, most preferably calcium chloride. Preferably, the kit contains sodium chloride, which may be combined with the clotting activator. The preferred concentration of sodium chloride solution is between 0.075M and 0.15M.

### EXAMPLE

An APTT performed with and without the inclusion of PCA reagent.
Method: Citrated blood is preferable but oxalate or EDTA may be used as an anticoagulant. Plasma is obtained by centrifugation at 3000g. We have performed the assay manually and in the APTT mode with the ACL instrument of Instrumentation Laboratory, Milan, Italy. Other commercial instruments may be used. The manual method will be described.
Method (i): Plasma (0.1 ml) is placed in a clotting tube at 37°C, followed by APTT reagent (Diagen micronised silica APTT reagent, Thame, Oxon, UK) (0.1 ml). The contents are mixed and incubated for 5 minutes. A mixture of 1 part 50mM calcium chloride and 1 part physiological (0.15M) saline (0.1 ml) is then added and the clotting time determined by the tilt tube method. This is the normal APTT; normal clotting times range from 30 sec to 45 sec. The test is repeated with the same APTT reagent containing PCA at 1.25.10⁻⁸M (Diagen PCA.APTT reagent). The normal clotting time will range from 65 to 300 seconds and when divided by the normal APTT, will give ratios of 2.0 to 10.0.
Method (ii): Plasma (0.1 ml) is placed in a clotting tube at 37°C followed by bovine thromboplastin (Diagen bovine thromboplastin, Thame, Oxon, UK) (0.1 ml). The contents are mixed and incubated for 5 minutes. 25mM calcium chloride (0.1 ml) is then added and the clotting time determined by the tilt tube method. This is the normal prothrombin time (PT); normal clotting times range from 27 sec. to 42 sec.. The test is repeated with the same bovine thromboplastin reagent containing PCA at 2.5.10⁻⁸M (Diagen bovine thromboplastin PCA reagent). The clotting time with PCA will range from 45 to 60 sec., and when divided by the normal PT will give ratios of 1.6 to 2.5.

Method (i) is to be preferred because of the greater increase in clotting times in the presence of PCA. This confers greater sensitivity in the detection of abnormality.

### EXPERIMENTAL RESULTS

Plasma samples from four patients with known APC cofactor defects, 57 normal plasma samples and 58 consecutive plasma samples from patients for thrombophilia screening, were tested with the PCA.APTT test and with the APC cofactor test of Chromogenix (Quadratech, Epsom, Surrey, UK). The limit of the abnormal range for both tests was set at a ratio of 2.1 or below. All four known APC cofactor defects gave ratios of less than 2.1 with both tests. Of the 57 normal plasma samples, three gave ratios of less than 2.1 with both tests, indicating a defect in the APC cofactor. Of the 58 patients screened for thrombophilia, three gave ratios of less than 2.1 with both tests, four gave ratios of less than 2.0 with the PCA.APTT test, but ratios of 2.1, 2.2, 2.4 and 2.6 with the APC.APTT test. All four showed normal PC. It was suspected that these four samples might be heterozygous for the APC cofactor defect; genomic DNA obtained from three of these patients has been subjected to polymerase chain reaction amplification and found to be heterozygous for the Factor V Leiden mutation. One patient sample gave a ratio of 2.5 with the APC.APTT test, but an abnormal PCA.APTT ratio of 1.7. Specific assay for PC showed that the sample had only 29% of average normal PC. All other abnormal samples had normal PC and PS by assay. A sample immuno-depleted in PS, gave an APC. APTT ratio of 1.7 and a PCA.APTT ratio of only 1.3. Thus the PCA method has been shown to detect all the APC cofactor defects detected by the APC method and also PC and PS. Samples which were normal, generally gave higher ratios with the PCA method, and seven out of nine abnormal samples gave lower ratios with the PCA method. This, together with the recognition of four extra abnormal samples may simply be reflecting a greater discrimination of the PCA test. The test should prove to be a useful test for APC resistance in routine hospital practice. The use of the PCA. APTT test may also serve to discover any other possible Factors or cofactors which may be involved in the endogenous formation of APC.

In methods according to the second aspect of the invention, when the patient is or may be receiving treatment for thrombophilia, the methodology is the same as for the general test, but the plasma sample is initially diluted from 1:5 to 1:10 in Factor V depleted plasma. The PCA.APTT/APTT or PCA.PT/PT ratio is then determined as for undiluted plasma; the results may be relied upon to give a good indicator of APC cofactor deficiency. Thus, in the preceding example, the 0.1 ml plasma sample would comprise 20 µl patient plasma and 80 µl Factor V depleted plasma.

## Claims

1. An APC cofactor deficiency test for determining whether a blood plasma sample in which protein C and protein S levels are normal by assay is deficient in APC cofactor, comprising:
(i) incubating a first portion of the sample with
(a) an exogenous reagent containing phospholipid which activates the contact mechanism in intrinsic clotting or an exogenous reagent which initiates extrinsic clotting; and incubating a second portion of the sample with
(b) the reagent of step (i)(a) and an exogenous Protein C (PC) activator which produces activated PC (APC) from endogenous Protein C and Protein S;
then (ii) adding to each of the two incubated portions a clotting agent and monitoring the conversion rate of fibrinogen to insoluble fibrin; and
(iii) calculating the ratio of the rate for the second portion of the sample to the rate for the first portion and comparing this ratio with the corresponding ratio for samples from normal subjects.

2. An APC cofactor deficiency test comprising steps (i) to (iii) of claim 1 in which the plasma sample is initially diluted with Factor V depleted plasma.

3. A test according to claim 2 in which the dilution of plasma sample in Factor V depleted plasma is from 1:5 to 1:10.

4. A test according to any preceding claim in which clotting agent added in step (ii) is a divalent or trivalent salt preferably calcium chloride.

5. A test according to any preceding claim in which the portions are incubated with an exogenous reagent, in which sodium chloride is added to the products of step(i) (a) and step (i)(b).

6. A test according to claim 5 in which the saline concentration in the products is between 0.075M and 0.15M.

7. A test according to any preceding claim in which the reagent of step (i) (a) is an APTT reagent.

8. A test according to any preceding claim in which the conversion rate monitored in step (ii) is monitored by clot formation.

9. A test according to any preceding claim in which the exogenous Protein C activator is any exogenous activator of Protein C or Protein S.

10. A test according to any preceding claim in which the reagents are in lyophilised form and are reconstituted with water or other diluent.

11. A kit for use in a test according to any preceding claim comprising:
an exogenous reagent containing phospholipid which activates the contact mechanism in intrinsic clotting or an exogenous,reagent which initiates extrinsic clotting; and, separately, the same exogenous reagent with an exogenous Protein C (PC) activator which produces activated PC (APC) from endogenous Protein C and Protein S; and
a clotting agent, preferably a divalent or trivalent salt, most preferably calcium chloride.

12. A kit according to claim 11 for use in a test according to claim 2 or 3 or to any of claims 4 to 10 when dependent from claim 2 or 3 further comprising Factor V depleted plasma.

13. A kit according to claim 11 or 12 in which at least one of the reagents is in lyophilised form.

14. A kit according to any of claims 11 to 13 which comprises an exogenous reagent further comprising sodium chloride solution.

15. A kit according to claim 14 in which the concentration of the sodium chloride is between 0.075M and 0.15M.

16. A test according to any of claims 1 to 10 or a kit according to any of claims 11 to 15 in which the exogenous reagent contains from 0.01 to 0.2%, preferably 0.04%, crude phospholipid.

17. A test according to any of claims 1 to 10 and 16 or a kit according to any of claims 11 to 15 in which the exogenous reagent is the supernatant after centrifuging of a 4% solution of acetone-dried bovine brain activated in saline solution.

18. A test according to any of claims 1 to 10, 16 and 17 or a kit according to any of claims 11 to 15 in which the concentration of PCA in the exogenous reagent containing PCA is from 0.25.10⁻⁸M to 2.5.10⁻⁸M, preferably 1.25.10⁻⁸M.

## Patentansprüche

1. APC-Kofaktormangeltest zum Bestimmen, ob eine Blutplasmaprobe, in der die Werte von Protein C oder Protein S nach Assay normal sind, einen Mangel an APC-Kofaktor hat, umfassend die folgenden Schritte:
(i) Inkubieren eines ersten Teils der Probe mit
(a) einem exogenen Reagens, das Phospholipid enthält, das den Kontaktmechanismus beim intrinsischen Gerinnen aktiviert, oder einem exogenen Reagens, das die extrinsische Gerinnung auslöst; und Inkubieren eines zweiten Teils der Probe mit
(b) dem Reagens aus Schritt (i)(a) und einem exogenen Protein-C-(PC)-Aktivator, der aktiviertes PC (APC) von endogenem Protein C und Protein S produziert;
anschließend (ii) Hinzufügen eines Gerinnungsmittels zu jedem der zwei inkubierten Teile und Überwachen der Umwandlungsgeschwindigkeit von Fibrinogen in unlösliches Fibrin; und
(iii) Berechnen des Verhältnisses zwischen der Geschwindigkeit beim zweiten Teil der Probe und der Geschwindigkeit beim ersten Teil und Vergleichen dieses Verhältnisses mit dem entsprechenden Verhältnis bei Proben von normalen Versuchsobjekten.

2. APC-Kofaktormangeltest, umfassend die Schritte (i) bis (iii), wobei die Plasmaprobe anfänglich mit Faktor-V-verarmtem Plasma verdünnt wird.

3. Test nach Anspruch 2, wobei die Verdünnung der Plasmaprobe in Faktor-V-verarmtem Plasma zwischen 1:5 und 1:10 liegt.

4. Test nach einem der vorherigen Ansprüche, wobei das in Schritt (ii) zugegebene Gerinnungsmittel ein zweiwertiges oder dreiwertiges Salz und vorzugsweise Calciumchlorid ist.

5. Test nach einem der vorherigen Ansprüche, wobei die Teile mit einem exogenen Reagens inkubiert werden, wobei Natriumchlorid in die Produkte aus Schritt (i)(a) und (i)(b) gegeben wird.

6. Test nach Anspruch 5, wobei die Salzlösungskonzentration in den Produkten zwischen 0,075 M und 0,15 M liegt.

7. Test nach einem der vorherigen Ansprüche, wobei das Reagens von Schritt (i)(a) ein APTT-Reagens ist.

8. Test nach einem der vorherigen Ansprüche, wobei die in Schritt (ii) beobachtete Umwandlungsgeschwindigkeit anhand der Gerinnselbildung beobachtet wird.

9. Test nach einem der vorherigen Ansprüche, wobei der exogene Protein-C-Aktivator irgendein exogener Aktivator von Protein C oder Protein S ist.

10. Test nach einem der vorherigen Ansprüche, wobei die Reagenzien in lyophilisierter Form vorliegen und mit Wasser oder einem anderen Verdünnungsmittel rekonstituiert werden.

11. Kit zur Verwendung in einem Test nach einem der vorherigen Ansprüche, umfassend:
ein exogenes Reagens, das Phospholipid enthält, das den Kontaktmechanismus beim intrinsischen Gerinnen aktiviert, oder ein exogenes Reagens, das die extrinsische Gerinnung auslöst; und, separat, das gleiche exogene Reagens mit einem exogenen Protein-C-(PC)-Aktivator, der aktiviertes PC (APC) von endogenem Protein C und Protein S produziert; und ein Gerinnungsmittel, das vorzugsweise ein zweiwertiges oder dreiwertiges Salz ist, wobei Calciumchlorid am meisten bevorzugt wird.

12. Kit nach Anspruch 11 zur Verwendung in einem Test nach Anspruch 2 oder 3 oder einem der Ansprüche 4 bis 10 in Abhängigkeit von Anspruch 2 oder 3, ferner umfassend Faktor-V-verarmtes Plasma.

13. Kit nach Anspruch 11 oder 12, wobei wenigstens eines der Reagenzien in lyophilisierter Form vorliegt.

14. Kit nach einem der Ansprüche 11 bis 13, umfassend ein exogenes Reagens, ferner umfassend Natriumchloridlösung.

15. Kit nach Anspruch 14, wobei die Konzentration des Natriumchlorids zwischen 0,075 M und 0,15 M liegt.

16. Test nach einem der Ansprüche 1 bis 10 oder Kit nach einem der Ansprüche 11 bis 15, wobei das exogene Reagens zwischen 0,01 und 0,2%, vorzugsweise 0,04% rohes Phospholipid enthält.

17. Test nach einem der Ansprüche 1 bis 10 und 16 oder Kit nach einem der Ansprüche 11 bis 15, wobei das exogene Reagens das Supernatant im Anschluss an die Zentrifugation einer 4%igen Lösung von acetongetrocknetem Rinderhirn ist, aktiviert in Salzlösung.

18. Test nach einem der Ansprüche 1 bis 10, 16 und 17 oder Kit nach einem der Ansprüche 11 bis 15, wobei die Konzentration von PCA in dem exogenen Reagens mit PCA zwischen 0,25 x 10⁻⁸ M und 2,5 x 10⁻⁸ M und vorzugsweise bei 1,25 x 10⁻⁸ M liegt.

## Revendications

1. Test de déficience en cofacteur APC pour déterminer si un échantillon de plasma sanguin dans lequel les niveaux de protéine C et de protéine S sont normaux par analyse est déficient en cofacteur APC, comprenant :
(i) l'incubation d'une première partie de l'échantillon avec
(a) un réactif exogène contenant un phospholipide qui active le mécanisme de contact en une coagulation intrinsèque ou un réactif exogène qui déclenche une coagulation extrinsèque ; et l'incubation d'une deuxième partie de l'échantillon avec
(b) le réactif de l'étape (i)(a) et un activateur de Protéine C (PC) exogène qui produit de la PC activée (APC) à partir de Protéine C et de Protéine S endogènes ;
puis (ii) l'addition à chacune des deux parties incubées d'un agent de coagulation et le contrôle de la vitesse de conversion du fibrinogène en fibrine insoluble ; et
(iii) le calcul du rapport de la vitesse de la deuxième partie de l'échantillon sur la vitesse de la première partie et la comparaison de ce rapport au rapport correspondant d'échantillons de sujets normaux.

2. Test de déficience en cofacteur APC comprenant les étapes (i) à (iii) de la revendication 1, dans lequel l'échantillon de plasma est dilué initialement avec du plasma appauvri en Facteur V.

3. Test selon la revendication 2, dans lequel la dilution de l'échantillon de plasma dans du plasma appauvri en Facteur V va de 1:5 à 1:10.

4. Test selon l'une quelconque des revendications précédentes, dans lequel l'agent de coagulation ajouté à l'étape (ii) est un sel divalent ou trivalent, de préférence du chlorure de calcium.

5. Test selon l'une quelconque des revendications précédentes, dans lequel les parties sont incubées avec un réactif exogène, dans lequel du chlorure de sodium est ajouté aux produits de l'étape (i)(a) et de l'étape (i)(b).

6. Test selon la revendication 5, dans lequel la concentration saline dans les produits se situe entre 0,075M et 0,15M.

7. Test selon l'une quelconque des revendications précédentes, dans lequel le réactif de l'étape (i)(a) est un réactif APTT.

8. Test selon l'une quelconque des revendications précédentes, dans lequel la vitesse de conversion contrôlée à l'étape (ii) est contrôlée par formation de caillots.

9. Test selon l'une quelconque des revendications précédentes, dans lequel l'activateur de Protéine C exogène est n'importe quel activateur exogène de Protéine C ou de Protéine S.

10. Test selon l'une quelconque des revendications précédentes, dans lequel les réactifs se présentent sous forme lyophilisée et sont reconstitués avec de l'eau ou un autre diluant.

11. Kit destiné à être utilisé dans un test selon l'une quelconque des revendications précédentes, comprenant :
un réactif exogène contenant un phospholipide qui active le mécanisme de contact en une coagulation intrinsèque ou un réactif exogène qui déclenche une coagulation extrinsèque ; et séparément, le même réactif exogène avec un activateur de Protéine C (PC) exogène qui produit de la PC activée (APC) à partir de Protéine C et de Protéine S endogènes ; et
un agent de coagulation, de préférence un sel divalent ou trivalent, de la manière la plus préférée du chlorure de calcium.

12. Kit selon la revendication 11 destiné à être utilisé dans un test selon la revendication 2 ou 3 ou l'une quelconque des revendications 4 à 10 dépendantes de la revendication 2 ou 3, comprenant en outre du plasma appauvri en Facteur V.

13. Kit selon la revendication 11 ou 12, dans lequel au moins un des réactifs se présente sous forme lyophilisée.

14. Kit selon l'une quelconque des revendications 11 à 13, qui comprend un réactif exogène comprenant en outre une solution de chlorure de sodium.

15. Kit selon la revendication 14, dans lequel la concentration de chlorure de sodium se situe entre 0,075M et 0,15M.

16. Test selon l'une quelconque des revendications 1 à 10 ou kit selon l'une quelconque des revendications 11 à 15, dans lequel le réactif exogène contient de 0,01 à 0,2 %, de préférence 0,04 %, de phospholipide brut.

17. Test selon l'une quelconque des revendications 1 à 10 et 16 ou kit selon l'une quelconque des revendications 11 à 15, dans lequel le réactif exogène est le produit surnageant après centrifugation d'une solution à 4% de cervelle bovine séchée à l'acétone activée dans une solution saline.

18. Test selon l'une quelconque des revendications 1 à 10, 16 et 17 ou kit selon l'une quelconque des revendications 11 à 15, dans lequel la concentration de PCA dans le réactif exogène contenant la PCA est de 0,25.10⁻⁸M à 2,5.10⁻⁸M, de préférence 1,25.10⁻⁸M.
